(19) Europäisches Patentamt
European Patent Office
Office européen des brevets

(11) **EP 1 421 841 B1**

(12) **EUROPÄISCHE PATENTSCHRIFT**

(45) Veröffentlichungstag und Bekanntmachung des Hinweises auf die Patenterteilung:
**08.08.2007 Patentblatt 2007/32**

(51) Int Cl.:
***A01D 41/127*** *(2006.01)*

(21) Anmeldenummer: **04100529.9**

(22) Anmeldetag: **22.10.2002**

(54) **Mähdrescher**

Combine harvester

Moissonneuse batteuse

(84) Benannte Vertragsstaaten:
**BE DE DK FR IT**

(30) Priorität: **25.10.2001 US 3884**

(43) Veröffentlichungstag der Anmeldung:
**26.05.2004 Patentblatt 2004/22**

(60) Teilanmeldung:
**07105930.7 / 1 811 293**

(62) Dokumentnummer(n) der früheren Anmeldung(en) nach Art. 76 EPÜ:
**02102471.6 / 1 305 994**

(73) Patentinhaber: **DEERE & COMPANY**
**Moline, Illinois 61265-8098 (US)**

(72) Erfinder:
• **Rains, Gerald, E.**
**Bettendorf,**
**IA 52722 (US)**
• **Phelan, James, Joseph**
**Bettendorf,**
**IA 52722 (US)**
• **Slavens, Zachary W.**
**Bettendorf,**
**IA 52722 (US)**
• **Kozicki, Andrzej**
**Milan,**
**IL 61264 (US)**
• **Funk, Robert, C.**
**Auburn,**
**IL 62615 (US)**

(74) Vertreter: **Holst, Sönke et al**
**Deere & Company,**
**European Office,**
**Patent Department**
**Steubenstrasse 36-42**
**68163 Mannheim (DE)**

(56) Entgegenhaltungen:
EP-A- 0 804 872    EP-A- 0 908 086
EP-A- 0 908 087    FR-A- 2 285 063
FR-A- 2 683 425    US-A- 5 561 250
US-A- 5 750 877

## Beschreibung

Die Erfindung betrifft einen Mähdrescher mit einem Korntank, einem Elevator zur Befüllung des Korntanks mit sauberem Korn und einem im Korntank angeordneten Kornfeuchtigkeitssensor, der eine Zelle aufweist, die durch eine Einlassöffnung mit vom Elevator abgegebenem Korn befüllbar ist.

Kornfeuchtigkeitssensoren werden in Mähdreschern verwendet, insbesondere in Präzisionslandwirtschaftsanwendungen. Kontinuierliche oder momentane (absetzliche) Kornfeuchtigkeitsmessungen erlauben einem Bediener, die Feuchtigkeit des Korns während des Erntens zu beobachten. In Verbindung mit einer Einrichtung zur Ortsbestimmung (Georeferenzierung), z. B. GPS, kann ein Feuchtigkeitssensor zur Feuchtigkeitskartierung verwendet werden. Außerdem werden Feuchtigkeitssensoren in Ertragskartierungsanwendungen genutzt. Wenn sie in Kombination mit einem Kornflusssensor benutzt werden, wird die Information des Feuchtigkeitssensors häufig verwendet, um die Masse trockenen Getreides anhand der Masse des feuchten Getreides zu berechnen. Diese Berechnung kann sowohl auf ein bestimmtes Volumen, auf eine Fläche oder eine Zeiteinheit bezogen sein und statisch oder dynamisch erfolgen.

In der EP 0 804 872 A wird ein Mähdrescher beschrieben, dessen Korntank durch einen Schneckenförderer befüllt wird. Am abgabeseitigen Ende des Schneckenförderers befindet sich ein Feuchtigkeitssensor, der von dem Korn umspült wird, das der Schneckenförderer abgibt. Als nachteilig ist anzusehen, dass der vom Feuchtigkeitssensor ausgegebene Messwert vom aktuellen Korndurchsatz, der Kontinuität des Kornflusses und von der Höhe des Korns über dem Sensor abhängt. Es sind daher aufwändige Maßnahmen zur durchsatzabhängigen Kompensation des Messwerts des Feuchtigkeitssensors zu treffen.

In der EP 0 908 086 A wird vorgeschlagen, einen Feuchtigkeitssensor seitlich am Körnerelevator anzubringen. Der Feuchtigkeitssensor hat in einer Ausführungsform eine verschwenkbar gelagerte Klappe, die zum Entleeren der Messzelle dient. Diese Ausführungsform ist relativ aufwändig, da drei Schwenklager erforderlich sind (eines für die Klappe am Gehäuse und zwei zwischen Gehäuse und Kolben bzw. Kolben und Klappe). Weiterhin ist diese Anordnung sehr verschleißbehaftet und störanfällig, da sich hinter den Klappen insbesondere feuchtes Erntegut leicht festsetzen und zu einer Blockierung des Schwenkmechanismus führen kann. In einer anderen Ausführungsform findet ein verschiebbarer Kolben Verwendung, der jedoch das Korn nicht zur Einlassöffnung, sondern zu einer separaten Auslassöffnung herausdrückt. Ein Nachteil besteht darin, dass durch die Anbringung außerhalb des Körnerelevators bei eventuellen Lecks des Feuchtigkeitssensors Korn verloren geht. Des Weiteren sind die Befülleigenschaften des Sensors insbesondere bei geringeren Erträgen ungünstig, da ein Teil des Erntegutstroms in eine Nebenanordnung umgeleitet wird und keine Zwangsbefüllung erfolgt.

Die US 5 327 708 A schlägt vor, unterhalb des Auslasses einer Korntankbefüllschnecke eines Mähdreschers einen zylindrischen, nach oben offenen Behälter anzubringen. An der Seitenwand des Behälters ist ein Feuchtigkeitssensor angeordnet. Der Boden des Behälters kann ferngesteuert geöffnet werden, so dass die Probe hinausfällt und neues Korn in den Behälter gelangen kann, um durch den Feuchtigkeitssensor untersucht zu werden. Insbesondere bei feuchtem Korn bleiben Reste der Probe (Korn und Verunreinigungen) an der Wand des Behälters hängen, so dass der Feuchtigkeitssensor mit der Zeit verschmutzt und ungenaue Messwerte abliefert. Durch die schwebende Aufhängung des Messzylinders gegenüber der Korntankbefüllschnecke kann es zusätzlich zu unterschiedlichen Befüllzeiten des Sensors kommen.

Die Firma TSI Montana bietet seit 1999 unter der Bezeichnung SCMS-2 einen selbstreinigenden Feuchtigkeitssensor für Mähdrescher an, der eine Messzelle mit einer Befüllöffnung aufweist. Ein Stößel transportiert das in der Messzelle angesammelte Korn wieder zur Befüllöffnung hinaus und reinigt dabei gleichzeitig die Messzelle. Dieser Feuchtigkeitssensor ist am Gehäuse eines Schneckenförderers anzubringen. Hier ist ebenfalls als nachteilig anzusehen, dass im Falle eventueller Lecks des Feuchtigkeitssensors Korn verloren gehen kann.

Die der Erfindung zu Grunde liegende Aufgabe wird darin gesehen, einen verbesserten Kornfeuchtigkeitssensor für einen Mähdrescher für die oben genannten Anwendungen bereitzustellen.

Diese Aufgabe wird erfindungsgemäß durch die Lehre des Patentanspruchs 1 gelöst, wobei in den weiteren Patentansprüchen Merkmale aufgeführt sind, die die Lösung in vorteilhafter Weise weiterentwickeln.

Der Kornfeuchtigkeitssensor wird innerhalb des Korntanks, beispielsweise versetzt zur Vorderseite des Übergangsgehäuses des Körnerelevators angeordnet. Ein Vorteil ist, dass ein Zugang zum Kornfeuchtigkeitssensor möglich ist, wenn erforderlich, und dass sich alle eventuellen Lecks der Messzelle im Korntank befinden, so dass kein Korn verloren gehen kann. Er weist eine Zelle auf, die aktiv (durch den Körnerelevator) mit Korn gefüllt wird. An dieser Stelle kann die Zelle unabhängig von (Hang-) Neigungsbedingungen des Mähdreschers gefüllt werden. Es erfolgt eine Zwangsbefüllung. Zur Befüllsteuerung der Messzelle ist ein (z. B. elektrisch, pneumatisch oder hydraulisch) fremdkraftbetriebener Aktor vorgesehen, der zwischen einer ersten und einer zweiten Position verschiebbar ist. In der ersten Position ermöglicht er das Füllen der Zelle mit Korn (bzw. füllt sie aktiv) und in der zweiten Position entleert er die Zelle.

Bei der im Folgenden beschriebenen Füllstandssensorplatte, der Abschirmung, der Messung der komplexen Leitwerte mittels eines Referenzleitwerts und der Messung der Ströme und Spannungen mit Synchrondetektoren handelt es sich um Merkmale, denen selbstständiger erfinderischer Rang zukommt.

In den Zeichnungen ist ein nachfolgend näher beschriebenes Ausführungsbeispiel der Erfindung dargestellt. Es zeigt:

Fig. 1A        eine Seitenansicht eines Mähdreschers mit einem erfindungsgemäßen Kornfeuchtigkeitssensor,

Fig. 1B        eine Seitenansicht eines erfindungsgemäßen Kornfeuchtigkeitssensors, der an einem Mähdrescher angebracht und in einer Füllposition ist,

Fig. 1C        eine Seitenansicht eines erfindungsgemäßen Kornfeuchtigkeitssensors, der an einem Mähdrescher angebracht und in einer Messposition ist,

Fig. 2        ein seitlicher Querschnitt durch die Zelle eines erfindungsgemäßen Kornfeuchtigkeitssensors,

Fig. 3        ein seitlicher Querschnitt durch die Zelle aus Figur 2, in der die Äquipotentiallinien des elektrischen Feldes dargestellt sind, das entsteht, wenn eine Anregungsspannung an die elektrisch beaufschlagte Platte angelegt wird,

Fig. 4        ein schematisches Schaltdiagramm eines Modells der erfindungsgemäßen Zelle,

Fig. 5        ein Blockdiagramm des Admittanzmeßkreises eines erfindungsgemäßen Kornfeuchtesensors, und

Fig. 6A und 6B        Blockdiagramme eines erfindungsgemäßen Feuchtesensorschaltkreises.

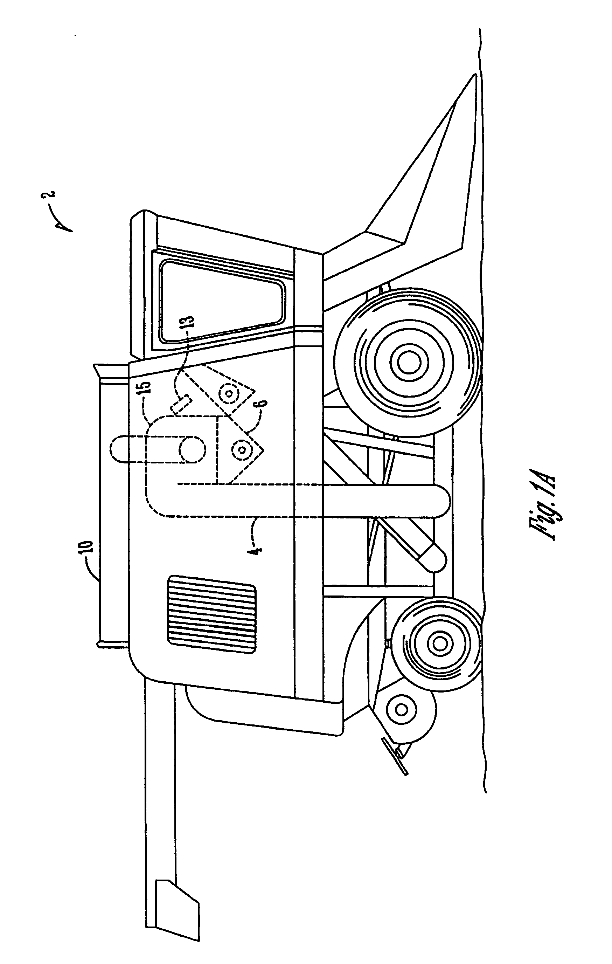

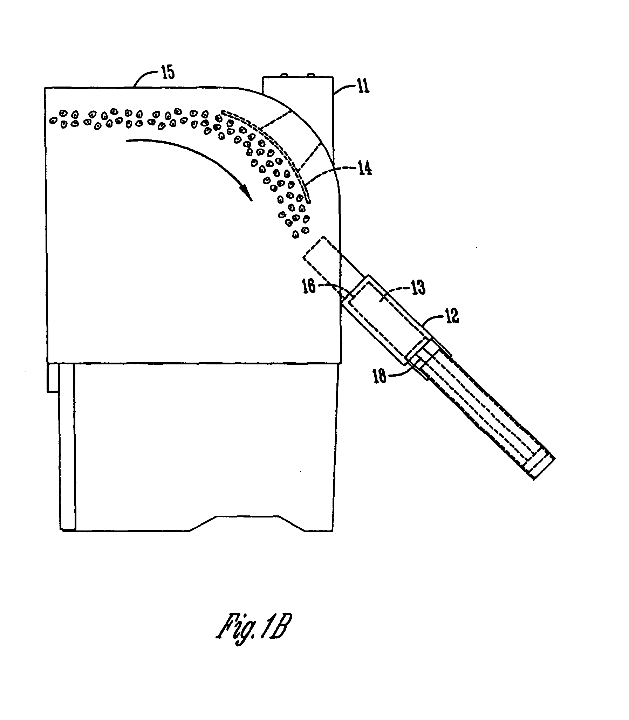

Die Figur 1A zeigt einen Mähdrescher mit einem erfindungsgemäßen Kornfeuchtigkeitssensor. In der Figur 1A ist der Mähdrescher 2 mit einem Korntank 10 dargestellt. Außerdem wird ein Elevator 4 für sauberes Korn gezeigt. Korn aus dem Elevator 4 für sauberes Korn bewegt sich zum Übergangsgehäuse 15 des Korntanks 10. Der Sumpf 6 des Übergangsgehäuses 15 wird auch gezeigt.

Die Figuren 1B und 1C zeigen Seitenansichten eines erfindungsgemäßen Kornfeuchtigkeitssensors, der an einem Mähdrescher angebracht ist. Der dargestellte Korntank 10 umfasst den Kornfeuchtigkeitssensor 12. Der Kornfeuchtigkeitssensor 12 ist im Korntank 10 des Mähdreschers 2 nahe dem Massenflusssensor 11 angeordnet. Die Öffnung 16 der Zelle 13 ist unterhalb der Prallplatte 14 im Übergangsgehäuse 15 angeordnet. Obwohl eine Prallplatte 14 dargestellt ist, beinhaltet die vorliegende Erfindung, dass andere Deflektoren verwendet werden können. An dieser Stelle wird die Zelle 13 aufgrund der direkten oder indirekten Geschwindigkeiten des Korns aktiv gefüllt, die durch die (nicht gezeigten) Paddel des Elevators 4 für sauberes Korn erzeugt werden. Dies erlaubt, dass die Zelle 13 mit hohen Raten gefüllt wird. Dies vermindert jegliche mit langsamen Zykluszeiten verbundene Probleme, die mit Bedingungen kleiner Flüsse verbunden sind, da hier die Zelle 13 wegen ihrer Anordnung innerhalb des vom Elevator 4 für sauberes Korn erzeugten Stroms mit einer hohen Rate gefüllt wird. Die Zelle 13 ist mit ihrem Einlass ausgerichtet. Ein Stößel mit Kolben 18 mit einem elektrischen Aktor wird verwendet, die Kornprobe zurück aus der Einlassöffnung 16 zu zwingen. In der Figur 1C sind die Zelle 13 und der Stößel mit Kolben 18 in einer Messposition.

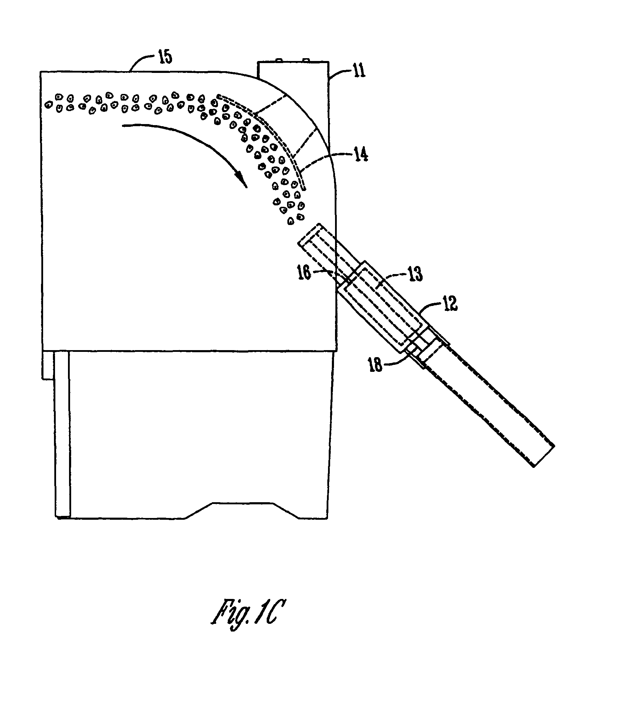

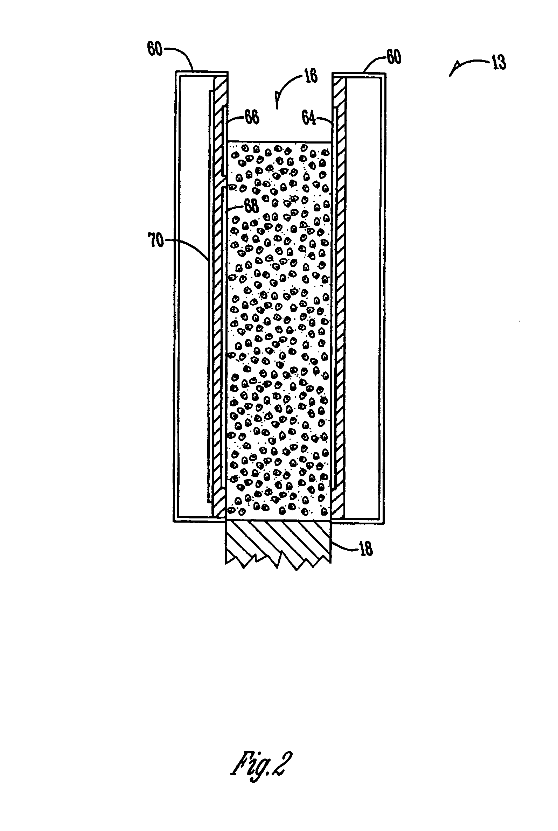

Die Figur 2 stellt eine Seitenansicht einer erfindungsgemäßen Zelle 13 dar. Die Öffnung 16 der Zelle 13 oder der Zwischenraum ist mit Korn gefüllt. An beiden Seiten des Zwischenraums sind parallele Kondensatorplatten 64 und 68. Die elektrisch beaufschlagte Platte 64 ist die Platte, an die eine Anregungsspannung angelegt ist. Die Sensorplatte 68 ist die Platte, an der der Strom gemessen wird, der die Zelle 13 durchströmt. Die Füllstandssensorplatte 66 ist nahe der Sensorplatte 68 und parallel zur elektrisch beaufschlagten Platte 64.

Die Füllstandssensorplatte 66 zur Erfassung des Füllstands der Zelle 13 ist von einem Viertel der Größe der Sensorplatte 68. Um festzustellen, ob oder wann die Zelle 13 gefüllt ist, sollte die Füllstandssensorplatte 66 einen Messwert bereitstellen, der ein Viertel des Messwerts der Sensorplatte 68 ist. Obwohl in dieser Ausführungsform die Füllstandssensorplatte 66 ein Viertel der Größe der Sensorplatte 68 hat, beinhaltet die vorliegende Erfindung verschiedene Änderungen in den Größen der Platten 64, 66 und 68. Dies ist nur ein Beispiel einer geeigneten und nützlichen relativen Größe.

Die Abschirmung 70 ist strategisch günstig hinter der Sensorplatte 68 und der Füllstandssensorplatte 66 angeordnet. Die Abschirmung 70 ist parallel zur Sensorplatte 68 und größer dimensioniert, um das elektrische Feld zu formen. Außerdem schirmt die Abschirmung 70 die Sensorplatte 68 von äußeren elektrischen Feldern ab, die von anderen Quellen als der elektrisch beaufschlagten Platte 64 erzeugt werden.

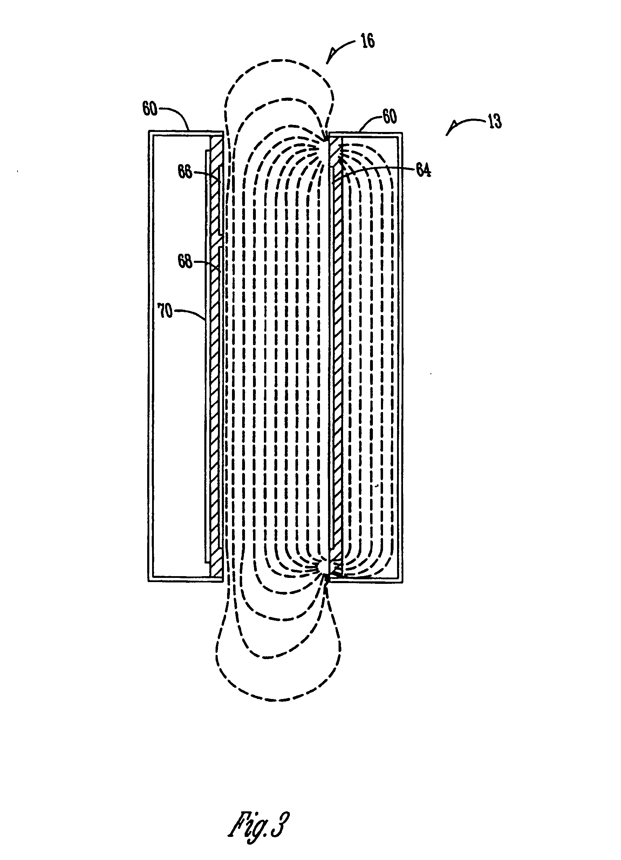

Die Figur 3 illustriert die Zelle 13 des Feuchtigkeitssensors 12 mit den Äquipotentiallinien des elektrischen Felds, das erzeugt wird, wenn ein Anregungssignal an die elektrisch beaufschlagte Platte 64 angelegt wird. Wegen der räumlichen Anordnung der Abschirmung 70, die von der Sensorplatte 68 elektrisch isoliert ist, aber auf demselben Potential liegt, ist die Wirkung auf die elektrischen Feldlinien in der Nachbarschaft der Sensorplatte 68, dass ein Äquivalent eines idealen Parallelplattenkondensators ohne Randeffekte erzeugt wird. Die elektrischen Feldlinien sind gerader Natur und senkrecht zur Sensorplatte 68 und zur Füllstandssensorplatte 66. Außerdem sind die elektrischen Feldlinien in der gesamten Region zwischen den parallelen Platten 66, 68 gleichförmiger Dichte. Das Ergebnis ist, dass Randeffekte vermindert sind. Randeffekte erzeugen unkontrollierbare Einflüsse auf die Messungen durch von Korn sich unterscheidende Materialien, die sich nahe aber außerhalb der Zelle 13 befinden. Hier zeigen die geraden elektrischen Feldlinien innerhalb der Zelle 13, dass die Zelle 13 im Wesentlichen gegen derartige Einflüsse immun ist. Außerdem stellt die gleichförmige elektrische Felddichte eine konstante Empfindlichkeit in der gesamten Zelle 13 bereit. Zusätzlich sind die ganze Zelle 13 und die Elektronik in einem Metallgehäuse 60 enthalten. Das Metallgehäuse 60 dient als Schirm gegen elektromagnetische Störungen und isoliert weiterhin die ganze Zelle 13 von anderen Quellen elektromagnetischer Energie.

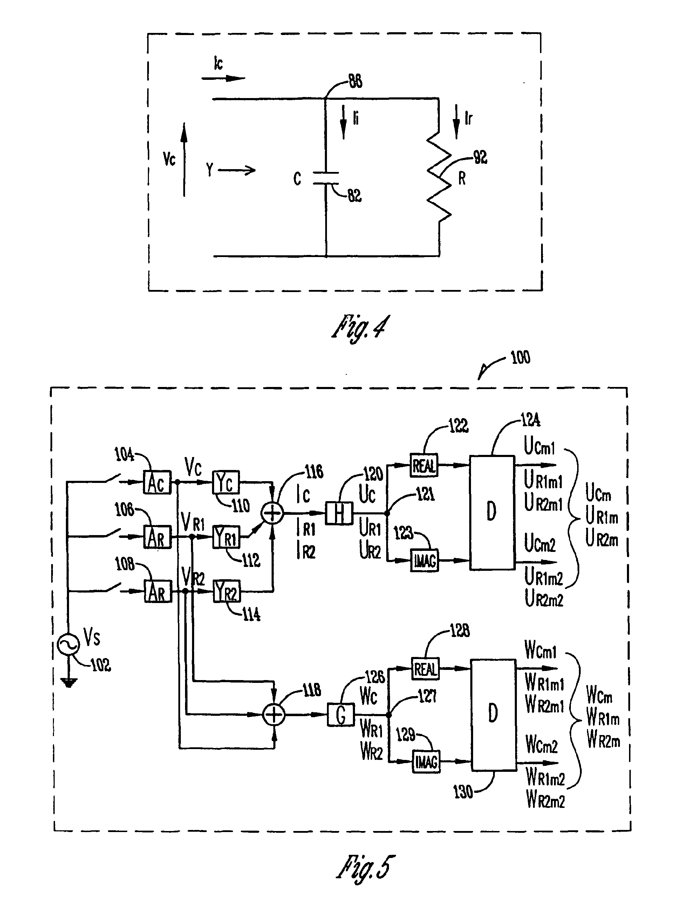

Die vorliegende Ausführungsform ermöglicht Kornfeuchtigkeitsmessungen basierend auf der Messung der komplexen relativen Dielektrizitätskonstante des Korns, die im Folgenden als komplexe Dielektrizitätskonstante bezeichnet wird. Die Figur 4 illustriert ein schematisches Diagramm eines Schaltkreises, der der Kondensatorzelle 13 elektrisch äquivalent ist. Diese Ersatzschaltung umfasst einen idealen Kondensator 82 mit einem Wert C, der parallel mit einem idealen Widerstand 92 mit dem Wert R geschaltet ist. Der ideale Kondensator 82 repräsentiert die kapazitive oder energiespeichernde Eigenschaft der Zelle 13 und der ideale Widerstand 92 repräsentiert die konduktive oder energiedissipative Eigenschaft der Zelle 13. C und R hängen von der Anregungsfrequenz und der Feuchtigkeit, Temperatur und bestimmten anderen Eigenschaften des Korns ab.

Der komplexe Leitwert der Zelle 13 ist

$$Y = (1/R) + j\omega C,$$

wobei $\omega = 2\pi f$, f die Anregungsfrequenz und j die imaginäre Einheit ist. Wenn die Zelle 13 leer ist, hat sie im Wesentlichen keine energiedissipativen Eigenschaften. Ihr Leitwert ist sehr nahe dem eines idealen Kondensators mit dem Wert $C_{CE}$:

$$Y_{CE} = j\omega C_{CE}.$$

Wenn die Zelle 13 mit Korn gefüllt ist, hat sie sowohl energiedissipative und energiespeichernde Eigenschaften. Ihr Leitwert ist

$$Y_{CF} = (1/R_{CF}) + j\omega C_{CF}.$$

Durch Teilen des Leitwerts der gefüllten Zelle 13 durch den der leeren Zelle 13 erhält man:

$$Y_{CF}/Y_{CE} = C_{CF}/C_{CE} - j/\omega C_{CE}R_{CE} == \varepsilon$$

Dieses Verhältnis ist die komplexe Dielektrizitätskonstante des Korns. Die komplexe Dielektrizitätskonstante ist eine intrinsische Materialeigenschaft und hängt nur von der Anregungsfrequenz und der Feuchtigkeit, Temperatur und bestimmter anderer Eigenschaften des Korns ab. Sie ist unabhängig von den Abmessungen und der Form der Zelle 13. Die komplexe Dielektrizitätskonstante wird im Allgemeinen folgendermaßen geschrieben:

$$\varepsilon = \varepsilon' - j\varepsilon'',$$

wobei $\varepsilon'$ die Dielektrizitätskonstante und $\varepsilon''$ der Verlustfaktor ist.

Es ist eine Aufgabe des Schaltkreises, den Leitwert der leeren Zelle 13 und der vollen Zelle 13 zu messen, um die obigen Gleichungen zu verwenden, um die komplexe Dielektrizitätskonstante des Korns zu berechnen. Wie in der Figur 4 gezeigt, wird die komplexe Anregungsspannung $V_C$ über den Schaltkreis gelegt. Der entstehende komplexe Strom $I_C$ fließt durch den Schaltkreis. $V_C$ hat eine reelle Komponente $V_r$ und eine imaginäre Komponente $V_i$:

$$V_C = V_r + j\ V_i$$

$I_C$ hat eine reelle Komponente $I_r$ und eine imaginäre Komponente Ii:

$$I_C = I_r + j\ I_i$$

Durch die Messung von $V_r$, $V_i$, $I_r$ und $I_i$ kann der komplexe Leitwert Y unter Verwendung komplexer Arithmetik berechnet werden:

$$Y = I_c/V_c = (I_r + j I_i) / (V_r + j V_i).$$

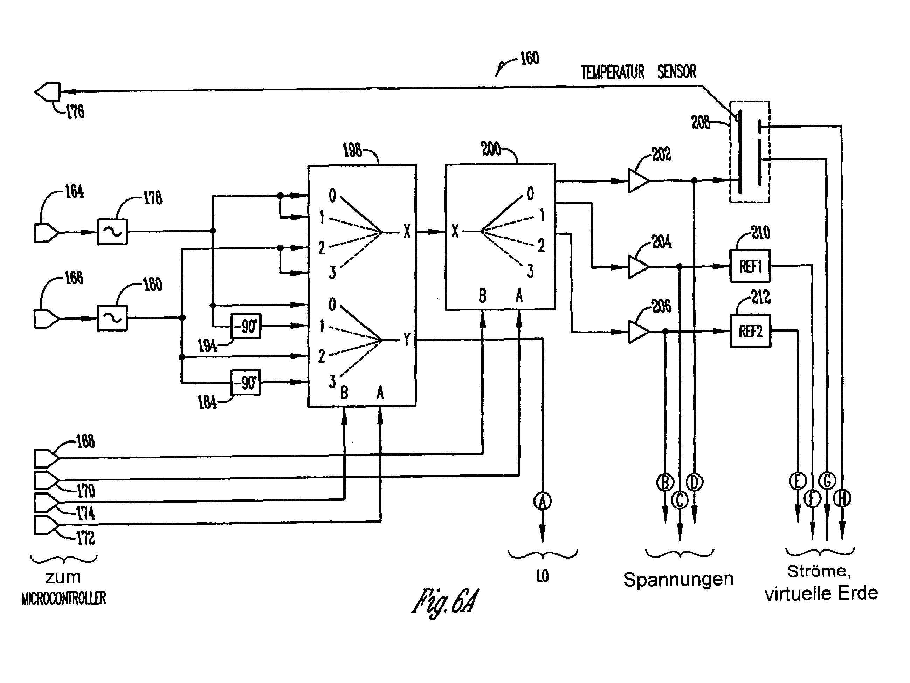

Die Figur 5 illustriert den im Kornfeuchtigkeitssensor verwendeten Schaltkreis zur Messung des Leitwerts. In der Figur 5 ist der Schaltkreis 100 zur Messung des Leitwerts gezeigt. Zu Erklärungszwecken werden die folgenden Definitionen verwendet:

$U_{R1m}$ wird als gemessener Wert des unten definierten $U_{R1}$ definiert.
$U_{R1m1}$ ist der Realteil (gleichphasiger Signalwert) von $U_{R1m}$.
$U_{R1m2}$ ist der Imaginärteil (Quadratur-Signalwert) von $U_{R1m}$.

$U_{R2m}$ wird als gemessener Wert des unten definierten $U_{R2}$ definiert.
$U_{R2m1}$ ist der Realteil (gleichphasiger Signalwert) von $U_{R2m}$.
$U_{R2m2}$ ist der Imaginärteil (Quadratur-Signalwert) von $U_{R2m}$.

$UC_m$ wird als gemessener Wert des unten definierten $U_C$ definiert.
$U_{Cm1}$ ist der Realteil (gleichphasiger Signalwert) von $A_{Cm}$.
$U_{Cm2}$ ist der Imaginärteil (Quadratur-Signalwert) von $A_{Cm}$.

$W_{R1m}$ wird als gemessener Wert des unten definierten $W_{R1}$ definiert.
$W_{R1m1}$ ist der Realteil (gleichphasiger Signalwert) von $W_{R1m}$.
$W_{R1m2}$ ist der Imaginärteil (Quadratur-Signalwert) von $W_{R1m}$.

$W_{R2m}$ wird als gemessener Wert des unten definierten $W_{R2}$ definiert.
$W_{R2m1}$ ist der Realteil (gleichphasiger Signalwert) von $W_{R2m}$.
$W_{R2m2}$ ist der Imaginärteil (Quadratur-Signalwert) von $W_{R2m}$.

$W_{Cm}$ wird als gemessener Wert des unten definierten $W_C$ definiert.
$W_{Cm1}$ ist der Realteil (gleichphasiger Signalwert) von $W_{Cm}$.
$W_{Cm2}$ ist der Imaginärteil (Quadratur-Signalwert) von $W_{Cm}$.

$U_C$ ist als komplexer Spannungswert definiert, der den die Zelle 13 durchfließenden Strom repräsentiert.
$U_{R1}$ ist als komplexer Spannungswert definiert, der den die erste Referenz durchfließenden Strom repräsentiert.
$U_{R2}$ ist als komplexer Spannungswert definiert, der den die zweite Referenz durchfließenden Strom repräsentiert.

$W_C$ ist als komplexer Spannungswert definiert, der die an der Zelle 13 anliegende Spannung repräsentiert.

$W_{R1}$ ist als komplexer Spannungswert definiert, der die an der ersten Referenz anliegende Spannung repräsentiert.
$W_{R2}$ ist als komplexer Spannungswert definiert, der die an der zweiten Referenz anliegende Spannung repräsentiert.

$I_C$ ist der komplexe, durch die Zelle fließende Strom.
$I_{R1}$ ist der komplexe, durch die erste Referenz fließende Strom.
$I_{R2}$ ist der komplexe, durch die zweite Referenz fließende Strom.

$V_C$ ist die komplexe Spannung an der Zelle.
$V_{R1}$ ist die komplexe Spannung an der ersten Referenz.
ist die komplexe Spannung an der zweiten Referenz.

$Y_C$ ist der komplexe Leitwert der Zelle.

$Y_{R1}$ ist der komplexe Leitwert der ersten Referenz.
$Y_{R2}$ ist der komplexe Leitwert der zweiten Referenz.

H ist die Übertragungsfunktion des Schaltkreises, der komplexe Strommessungen durchführt.
G ist die Übertragungsfunktion des Schaltkreises, der komplexe Spannungsmessungen durchführt.
$V_S$ ist die erzeugte Quellenspannung.

$A_C$ ist die Übertragungsfunktion der Zellenbetriebsspannung.
$A_R$ ist die Übertragungsfunktion der Referenzbetriebsspannung.

D ist die Übertragungsfunktion der Phasen- und Gewinnfehlanpassung zwischen den gemessenen reellen (phasengleichen) und gemessenen imaginären (Quadratur-) Komponenten des komplexen Stroms und der Spannung. Diese Fehlanpassung wird durch Fehler in den Schaltkreiselementen bedingt, die die Messung durchführen. D ist auch als "Mischertransformationsmatrix" bekannt. Der Wert von D ist zu messen und sein Einfluss auszugleichen.

Im Schaltkreis 100 zur Messung des Leitwerts wird eine erzeugte Quellspannung 102 ($V_S$) selektiv an die Zelle 13 oder an eine einer Mehrzahl von Referenzen über eine zugeordnete Übertragungsfunktion angelegt, wie durch die Bezugszeichen 104, 106 und 108 dargestellt. Wenn $V_S$ an die Übertragungsfunktion $A_C$ 104 angelegt wird, wird eine Spannung $V_C$ erzeugt, die an den komplexen Leitwert der Zelle $Y_C$ 110 angelegt wird. Wenn analog die Spannung 102 ($V_S$) an eine erste Übertragungsfunktion $A_R$ 106 angelegt wird, wird die entstehende Spannung $V_{R1}$ an den komplexen Leitwert des ersten Referenzleitwerts $Y_{R1}$ 112 angelegt, und wenn das Signal 102 an die zweite Übertragungsfunktion $A_R$ 108 angelegt wird, wird die entstehende Spannung $V_{R2}$ an den zweiten komplexen Leitwert 114 ($Y_{R2}$) angelegt. Jeder der entstehenden Ströme wird in einem Addierer 116 summiert. Wenn nur ein Pfad ausgewählt wird, wird nur eines dieser Signale nicht Null sein. Der sich ergebende Strom ist dann $I_C$, wenn die Zelle ausgewählt wird, $I_{R1}$ wenn die erste Referenz ausgewählt wird, und $I_{R2}$ wenn die zweite Zelle ausgewählt wird. Der entstehende Strom fließt durch einen Schaltkreis mit einer Übertragungsfunktion H 120, wobei H eine Übertragungsfunktion zur Umsetzung komplexen Stroms in eine komplexe Spannung für Messzwecke ist. Die entstehende, durch den Knoten 121 gemessene Spannung repräsentiert den komplexen Strom durch entweder den Leitwert der Zelle 13 oder einen der Referenzleitwerte. Die reellen (phasengleichen) und imaginären (Quadratur-) Komponenten dieser Spannung werden festgestellt, indem die Spannung an den Unterschaltkreis angelegt wird, der aus den Blöcken 122, 123 und 124 besteht, wie in Figur 5 gezeigt. Auf diese Weise werden daher Spannungen $U_{cm1}$ und $U_{cm2}$ gemessen, die den komplexen Strom durch die Zelle repräsentieren. Durch das Auswählen je einer der Referenzen können auch Spannungen gemessen werden, die den komplexen Strom durch die erste Referenz oder die zweite Referenz repräsentieren.

Zusätzlich zum Messen von Spannungen, die die komplexen Stromwerte repräsentieren, werden entsprechend des Schaltkreises auch Spannungen berechnet, die die komplexen Spannungswerte repräsentieren. Die Spannungen von der Zelle, $V_C$, der ersten Referenz, $V_{R1}$, und der zweiten Referenz, $V_{R2}$, werden an einen Addierer 118 angelegt. Da nur eine der Referenzen oder die Zelle gleichzeitig ausgewählt werden, wird nur einer dieser Werte von Null verschieden sein. Das Ergebnis wird an eine Übertragungsfunktion 126 angelegt, die eine komplexe Spannung am Knoten 127 ergibt. Die reellen und imaginären (phasengleichen und Quadratur) Komponenten dieser Spannung werden durch Anlegen der Spannung an den Unterschaltkreis festgestellt, der aus den Blöcken 128, 129 und 130 besteht, wie in Figur 5 dargestellt.

Auf diese Weise ermöglicht der in Figur 5 dargestellte Schaltkreis eine Bestimmung der Real- und Imaginärteile sowohl der Spannung als auch des Stroms, der bzw. die mit einem ausgewählten Leitwert verbunden ist oder sind. Dieser Leitwert ist entweder mit der Zelle 13 des Kornfeuchtigkeitssensors 12 oder mit einer der Referenzleitwerte des Kornfeuchtigkeitssensors verbunden.

Zur weiteren Erklärung liegen die folgenden mathematischen Beziehungen vor:

$$G = W/V$$

$$H = U/I$$

In jedem Fall sind die jeweiligen Übertragungsfunktionen als Verhältnis des Ausgangswerts der Funktion zum Eingangswert der Funktion definiert. Außerdem ist der Leitwert mathematisch definiert als:

$$Y = I/V = (U/H)/(W/G) = (U/W) \cdot (G/H).$$

Sind diese allgemeinen Beziehungen gegeben, ist der Leitwert einer Referenz definiert als:

$$Y_R = U_R/W_R \cdot G/H.$$

Außerdem werden die Leitwerte der leeren Zelle 13, $Y_{CE}$, und einer vollen Zelle, $Y_{CF}$, folgendermaßen berechnet:

$$Y_{CE} = U_{CE}/W_{CE} \cdot G/H.$$

$$Y_{CF} = U_{CF}/W_{CF} \cdot G/H.$$

Wenn die Messungen für den Referenzleitwert und den Leitwert der Zelle bei denselben Umgebungsbedingungen durchgeführt werden, kann angenommen werden, dass sowohl H als auch G in den Leitwertgleichungen für die Zelle und die Referenzen gleich sind. Dann kennzeichnet das Folgende die Kalibrierfaktoren für die leere Zelle und die Referenzen:

$$W_R/U_R \cdot Y_R = W_{CE}/U_{CE} \cdot Y_{CE} \Rightarrow F = W_{CE}/U_{CE} \cdot U_R/W_R = Y_R/Y_{CE}$$

Der Kalibrierfaktor F der Referenz gibt das Verhältnis vom Leitwert der Referenz zum Leitwert der leeren Zelle bei denselben Umgebungsbedingungen wieder. Daher kann zu Kalibrierzwecken ein
Referenzleitwert anstelle eines Leitwerts der leeren Zelle verwendet werden.
Unter der Annahme, dass F konstant bleiben wird, kann die komplexe Dielektrizitätskonstante der Kornprobe berechnet werden zu:

$$\varepsilon = (U_{CF} \cdot W_R)/(W_{CF} \cdot U_R) \cdot F,$$

wobei

$$\varepsilon = \varepsilon' - j\,\varepsilon''.$$

Daher ermöglicht die vorliegende Ausführungsform eine Messung des komplexen Leitwerts des Korns zu Feuchtigkeitsmesszwecken.

Um genaue Strom- und Spannungsmessungen durchzuführen, ist es zweckmäßig, dass die phasengleichen (IP) und Quadratur- (Q) Signale der lokalen Oszillatoren, die bei den Mischern 216, 220 und 224 zum Extrahieren der reellen und imaginären Komponenten komplexer Signale verwendet werden, eine Phasendifferenz von genau 90 Grad und an ihren Grundfrequenzen identische Amplituden haben. Fehler werden in dem Maß eingeführt, in dem das nicht der Fall ist. Durch die Verwendung zweier Referenzleitwerte mit bekannten und stabilen Werten werden jedoch Korrekturen dieser Fehler durchgeführt.

Die D-Funktionen 124 und 130 repräsentieren die Verzerrung des Imaginärteils gegenüber dem Realteil aller gemessener komplexer Werte. Alle gemessenen Werte $U_m$ und $W_m$ können durch Verwendung derselben Formel korrigiert werden, um U und W zu erhalten, welches die Werte sind, bevor jegliche Messwertverzerrfehler eingeführt werden.

Folgendes ist die verzerrte Beziehung zwischen den komplexen Spannungen, die die Ströme durch die Zelle und die Referenzen und ihre gemessenen Werte repräsentieren:

$$U = [1\ \ j]\cdot D^{-1}\cdot U_m,$$

wobei

$$D^{-1} = PFC = \begin{matrix} 1 & 0 \\ Pfc1 & pfc2 \end{matrix}$$

$$U_m = \begin{matrix} U_{m1} \\ U_{m2} \end{matrix}$$

Dieselbe verzerrte Beziehung gilt zwischen den komplexen Spannungen, die die Zell- und Referenzspannungen und ihre gemessenen Werte repräsentieren:

$$W = [1\ \ j]\cdot D^{-1}\cdot W_m,$$

wobei

$$D^{-1} = PFC = \begin{matrix} 1 & 0 \\ Pfc1 & pfc2 \end{matrix}$$

$$W_m = \begin{matrix} W_{m1} \\ W_{m2} \end{matrix}$$

Das Expandieren der obigen Gleichungen ergibt:

$$U = U_{m1} + j \cdot (pfc1 \cdot U_{m1} + pfc2 \cdot U_{m2})$$

$$W = W_{m1} + j \cdot (pfc1 \cdot W_{m1} + pfc2 \cdot W_{m2})$$

Die Korrekturfaktoren pfc1 und pfc2 werden durch die Verwendung zweier unterschiedlicher Referenzen herausgefunden, die bekannte und stabile Leitwerte mit verschiedenen Phasenwinkeln haben. Als Beispiel ist in einer Ausführungsform erste Referenzwert ein temperaturstabiler Kondensator mit 1 % Toleranz (COG) mit einem Wert von 15 pF (Leitwert $Y_{R1}$) und die zweite Referenz ein Präzisionswiderstand mit 0,1 % Toleranz mit einem Wert von 2000 Ω (Leitwert $Y_{R2}$). Auch andere Referenzwerte können genutzt werden.
Das Verhältnis der Referenzleitwerte wird folgendermaßen berechnet:

$$R = Y_{R1}/Y_{R2} = Q_R + j \cdot Q_I.$$

Das Verhältnis der rohen Messwerte der zwei Referenzen ist:

$$R_m = (U_{R1} \cdot W_{R2}) / (W_{R1} \cdot U_{R2}).$$

In die obige Gleichung können die Ergebnisse der Messungen eingefügt werden, so dass man erhält:

$$R_m = ((U_{R1m1}+j(U_{R1m1} \cdot pfc1+U_{R1m2} \cdot pfc2))(W_{R2m1}+j(W_{R2m1} \cdot pfc1+W_{R2m2} \cdot pfc2)))$$
$$/ ((W_{R1m1}+j(W_{R1m1} \cdot pfc1+W_{R1m2} \cdot pfc2))(U_{R2m1}+j(U_{R2m1} \cdot pfc1+U_{R2m2} \cdot pfc2))).$$

$R_m$ wird mit R gleichgesetzt und zwei quadratische Gleichungen mit zwei Unbekannten (pfc1, pfc2) werden abgeleitet:

$$a_1 \cdot pfc1^2 + b_1 \cdot pfc2^2 + c_1 \cdot pcf1 \cdot pfc2 + d_1 \cdot pfc1 + e_1 \cdot pfc2 + f_1 = 0$$

(vom Realteil)

$$a_2 \cdot pfc1^2 + b_2 \cdot pfc2^2 + c_2 \cdot pcf1 \cdot pfc2 + d_2 \cdot pfc1 + e_2 \cdot pfc2 + f_2 = 0$$

(vom Imaginärteil),
wobei

$$a_1 = Q_R \; W_{R1m1} \cdot U_{R2m1} - U_{R1m1} \cdot W_{R2m1}$$

$$a_2 = Q_I \; W_{R1m1} \cdot U_{R2m1}$$

$$b_1 = Q_R \; W_{R1m2} \cdot U_{R2m2} - U_{R1m2} \cdot W_{R2m2}$$

$$b_2 = Q_I \; W_{R1m2} \cdot U_{R2m21}$$

$$c_1 = Q_R \; W_{R1m1} \cdot U_{R2m2} + Q_R \cdot W_{R1m2} \cdot U_{R2m1} - U_{R1m1} \cdot W_{R2m2} - U_{R1m2} \cdot W_{R2m1C}$$

$$c_2 = e_1$$

$$d_1 = 2 \cdot Q_I \; W_{R1m1} \cdot U_{R2m1}$$

$$d_2 = -2 \cdot a_1$$

$$e_1 = Q_I \cdot ( \; W_{R1m1} \cdot U_{R2m2} + W_{R1m2} \cdot U_{R2m1)}$$

$$e_2 = - c_1$$

$$f_1 = - a_1$$

$$f_2 = - a_2$$

Diese zwei quadratischen Gleichungen werden dann gleichzeitig für pfc1 und pfc2 gelöst. Da eine einfache, geschlossene Form nicht vorhanden ist, können sie beispielsweise durch Newton-Raphson-Iteration gelöst werden. Andere Algorithmen zur Lösung numerischer Gleichungen können ebenfalls verwendet werden. Es ist bekannt, dass die Lösung nahe des Punktes (pcf1=0, pfc2=1) liegt, so dass diese vorzugsweise als Startpunkt verwendet werden. Theoretisch sind vier verschiedene Lösungen möglich. Jede Lösung, die nicht nahe (0,1) liegt, sollte als ungeeignet angesehen werden. Bei einer Softwareimplementation kann eine geeignete Fehlerbedingung gesetzt werden. Es ist nicht wahrscheinlich, dass dies vorkommt, jedoch können Vorsichtsmaßnahmen für den Fall, dass es vorkommt, vorgesehen werden.

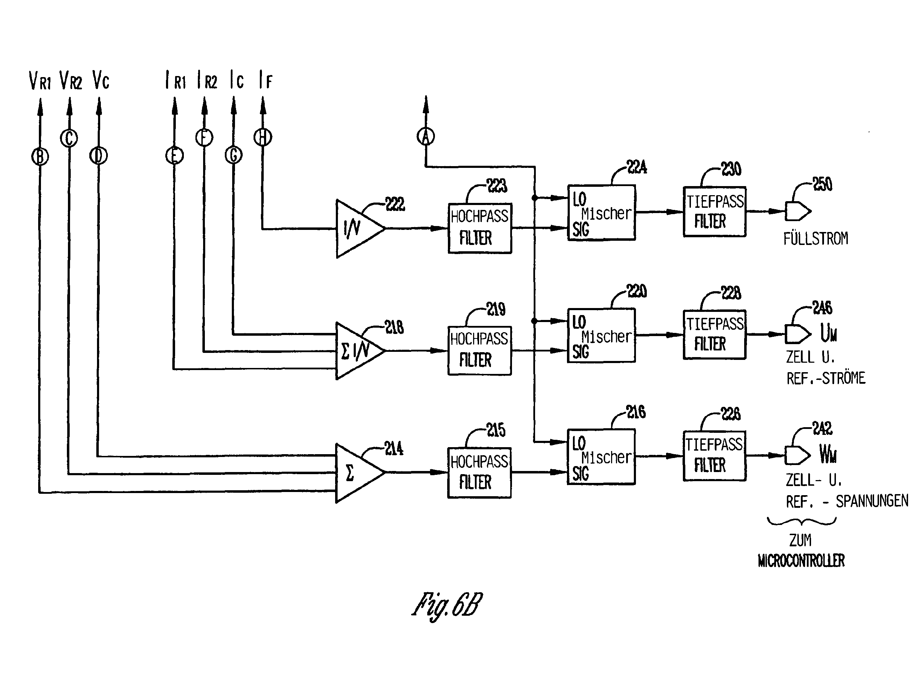

Die Figuren 6A und 6B zeigen ein Schema für den Kornfeuchtigkeitssensor. Das Schema zeigt eine Anzahl an Eingangs- und Ausgangsleitungen zur Verbindung mit einer intelligenten Steuerung, wie einem Prozessor, Mikrocontroller, integriertem Schaltkreis oder anderem Gerät. Dieses Schema zeigt nur eine Schaltkreiskonfiguration. Die vorliegende Ausführungsform stellt die Möglichkeit bereit, selektiv eine von einer Mehrzahl komplexer Leitwerte bei einer Vielzahl von Frequenzen zu messen.

Die Eingänge in das System (Ausgänge einer intelligenten Steuerung) werden in Figur 6A gezeigt. Die Eingänge umfassen einen ersten Frequenzeingang 164 und einen zweiten Frequenzeingang 166. Optional werden ein erster Sinuswellengenerator 178 und ein zweiter Sinuswellengenerator 180 verwendet. Die Sinuswellengeneratoren nehmen den Rechteckwellenausgang eines Mikrocontrollers, teilen die Frequenz wie erforderlich und glätten den Ausgang, so dass ein sinusförmiges Signal erzeugt wird. Der Ausgang des ersten Sinuswellengenerators 178 ist elektrisch mit drei Schaltereingängen eines doppelten Umschalters 198 mit vier Eingängen verbunden. Zusätzlich ist der Ausgang des ersten Sinuswellengenerators 178 elektrisch mit einem 90° Phasenschieber 194 verbunden. Der 90° Phasenschieber 194 ist derart aufgebaut, dass sein Ausgangssignal um 90° in der Phase gegenüber seinem Eingangssignal verschoben ist. Der 90° Phasenschieber ist elektrisch mit dem Schaltereingang des doppelten Umschalters 198 verbunden. Der Ausgang des zweiten Sinuswellengenerators 180 ist ähnlich angeschlossen.

Der erste Sinuswellengenerator 178 und der zweite Sinuswellengenerator 180 arbeiten bei unterschiedlichen Frequenzen. Beispielsweise arbeitet der erste Sinuswellengenerator 178 bei 10 MHz, während der zweite Sinuswellengenerator bei 1 MHz arbeitet.

Der doppelte Umschalter 198 wird durch die Eingänge 172 und 174 gesteuert, die verwendet werden, eines der Signale auszuwählen. Einer der Ausgänge des Schalters 198 ist elektrisch mit einem Eingang des doppelten Umschalters 200 mit zwei Ausgängen verbunden. Eingänge 168 und 170 sind mit dem Umschalter 200 verbunden, um zu steuern, welcher der Ausgänge ausgewählt wird. Die Ausgänge sind gepuffert und dann elektrisch mit der Sensorzelle 208 (die der Zelle 13 in den anderen Figuren entspricht), einem ersten Referenzleitwert 210 und einem zweiten Referenzleitwert 212 verbunden. Die Referenzleitwerte werden für Kalibrierzwecke verwendet.

Wie in Figur 6B gezeigt, sind die gepufferten Ausgänge, die die Zelle 208 und die beiden Referenzen treiben, auch elektrisch mit einem Summierkreis 214 verbunden. Der Ausgang des Summierkreises ist elektrisch über einen Hochpassfilter 215 mit einem Mischer 216 verbunden. Der Mischer 216 hat auch einen lokalen Oszillatoreingang, der elektrisch mit einem Ausgang des Schalters 198 (Figur 6A) verbunden ist. Der Ausgang des Mischers 216 geht durch einen Tiefpassfilter 226 und ist dann elektrisch mit einem Analog/Digitalwandler verbunden und wird durch den Mikrocontroller ausgelesen. Am Ausgang des Mischers 216 liegt eine Gleichspannung an, die proportional zur Komponente des Eingangsspannungssignals ist, welche mit dem lokalen Oszillator gleichphasig ist.

Die Sensorplatte 68 der Zelle 208 und die erste Referenz 210 und die zweite Referenz 212 aus Figur 6A sind elektrisch mit einem summierenden Strom/Spannungsumsetzer 218 verbunden, wie er in Figur 6B gezeigt ist. Der summierende Strom/Spannungsumsetzer 218 hat einen Eingang mit niedriger Impedanz, der virtuell auf Erdpotential liegt. Der Ausgang des summierenden Strom/Spannungsumsetzers 218 ist über ein Hochpassfilter 219 elektrisch mit einem zweiten Mischer 220 verbunden. Der zweite Mischer 220 hat auch einen lokalen Oszillatoreingang, der elektrisch mit einem Ausgang des Schalters 198 (Figur 6A) verbunden ist. Der Ausgang des Mischers 220 geht durch einen Tiefpassfilter 228 und ist dann elektrisch mit einem Analog/Digitalwandler verbunden und wird durch den Mikrocontroller ausgelesen. Am Ausgang des Mischers 220 liegt eine Gleichspannung an, die proportional zur Komponente des Eingangssignals ist, welches mit dem lokalen Oszillator gleichphasig ist.

Außerdem läuft der Strom $I_F$ von der Füllstandssensorplatte 66 an der Zelle 208 (s. Figur 6A) durch den Strom/Spannungsumsetzer 222. Dieser Strom/Spannungsumsetzer 222 hat ebenfalls einen Eingang mit niedriger Impedanz, der sich virtuell auf Erdpotential befindet. Der Ausgang des Strom/Spannungsumsetzers 222 ist über ein Hochpassfilter 223 mit einem dritten Mischer 224 verbunden. Der dritte Mischer 224 hat auch einen lokalen Oszillatoreingang, der elektrisch mit einem Ausgang des Schalters 198 verbunden ist. Der Ausgang des Mischers 224 geht über einen Tiefpassfilter 230 an einen Analog/Digitalwandler und wird vom Mikrocontroller ausgelesen. Diese Konfiguration ermöglicht eine Überwachung des Leitwerts der Füllstandssensorplatte 66 gegenüber der Sensorplatte 68. Wenn dieses Verhältnis proportional zu den relativen Größen der Platten 66, 68 ist, dann wird die Zelle 208 als mit Korn gefüllt angesehen.

Das synchrone Nachweisverfahren der Messung komplexer Signale unter Verwendung eines lokalen Oszillators, eines Mischers und eines Tiefpassfilters, wie es oben beschrieben wurde, hat einen sehr engen Bandpassfiltereffekt, der den Untergrundgeräuscheinfluss auf die Messung wesentlich reduziert. Das Verfahren der Messung sehr kleiner Ströme mit virtueller Erde wird genutzt, um den Vorteil einer wesentlichen Verminderung des Einflusses parasitärer Elemente auf den Stromsummier- und Messknoten zu erzielen.

In der Figur 6A ist ein Thermistor oder ein anderer Temperatursensor an der elektrisch beaufschlagten Platte 64 der Zelle 208 angebracht. Dies ist nur ein Beispiel der Anbringung eines Temperatursensors. Der Temperatursensor kann auch an einer der anderen Platten der Zelle 208 angebracht sein. Die Messung der Temperatur erlaubt eine entsprechende Korrektur der Feuchtigkeitsberechnungen.

**Patentansprüche**

1. Mähdrescher (2) mit einem Korntank (10), einem Elevator (4) zur Befüllung des Korntanks (10) mit sauberem Korn und einem im Korntank (10) angeordneten Kornfeuchtigkeitssensor (12), der eine Zelle (13) aufweist, die durch eine Einlassöffnung (16) mit vom Elevator (4) abgegebenem Korn befüllbar ist, wobei die Zelle (13) innerhalb des vom Elevator (4) abgegebenen Stromes des Korns angeordnet und damit befüllbar ist, **dadurch gekennzeichnet, dass** die Zelle (13) einen Stößel mit einem verschiebbaren Kolben (18) aufweist, der betreibbar ist, eine Kornprobe aus der Einlassöffnung (16) zurückzuzwingen.

2. Mähdrescher (2) nach Anspruch 1, **dadurch gekennzeichnet, dass** der Kolben (18) zwischen einer ersten Position, in der er das Befüllen der Zelle (13) mit Korn ermöglicht, und einer zweiten Position bewegbar ist, in der er die Zelle (13) entleert.

3. Mähdrescher (2) nach einem der Ansprüche 1 oder 2, **dadurch gekennzeichnet, dass** der Kolben (18) durch einen elektrischen Aktor bewegbar ist.

4. Mähdrescher (2) nach einem der vorhergehenden Ansprüche, **dadurch gekennzeichnet, dass** der Kornfeuchtigkeitssensor (12) in einem Übergangsgehäuse (15) des Korntanks (10) stromab einer Prallplatte (14) angeordnet ist, die derart angeordnet ist, dass sie im Betrieb den vom Elevator (4) abgegebenen Kornfluss in die Zelle (13) umlenkt.

**Claims**

1. Combine harvester (2) having a grain tank (10), an elevator (4) for filling the grain tank (10) with clean grain and a grain moisture sensor (12), which is arranged in the grain tank (10) and has a cell (13), which can be filled with grain delivered by the elevator (4) through an inlet opening (16), the cell (13) being arranged within the flow of grain delivered by the elevator (4) and being capable of being filled with it, **characterized in that** the cell (13) has a plunger having a displaceable piston (18), which can be operated so as to force a grain sample back out of the inlet opening (16).

2. Combine harvester (2) according to Claim 1, **characterized in that** the piston (18) is capable of moving between a first position, in which it makes it possible for the cell (13) to be filled with grain, and a second position, in which it empties the cell (13).

3. Combine harvester (2) according to either of Claims 1 and 2, **characterized in that** the piston (18) is capable of being moved by an electrical actuator.

4. Combine harvester (2) according to one of the preceding claims, **characterized in that** the grain moisture sensor (12) is arranged in a transition housing (15) of the grain tank (10) downstream of a baffle plate (14), which is arranged such that, during operation, it deflects the flow of grain delivered by the elevator (4) into the cell (13).

**Revendications**

1. Moissonneuse-batteuse (2) comportant une trémie à grains (10), un élévateur (4) pour acheminer des grains propres dans la trémie à grains (10) et un capteur d'humidité des grains (12), qui est disposé dans la trémie à grains (10) et qui comporte une cellule (13), qui peut être remplie par les grains déversés par l'élévateur (4) par l'intermédiaire d'une ouverture d'admission (16), la cellule (13) étant disposée à l'intérieur du flux de grains distribué par l'élévateur (4) et pouvant être remplie par celui-ci, **caractérisée en ce que** la cellule (13) comporte un poussoir avec un piston (18) mobile en translation, qui est destiné à être utilisé pour repousser un échantillon de grains à travers l'ouverture d'admission (16).

2. Moissonneuse-batteuse (2) selon la revendication 1, **caractérisée en ce que** le piston (18) peut être déplacé entre une première position, dans laquelle il permet le remplissage de la cellule (13) avec les grains, et une deuxième position, dans laquelle il vide la cellule (13).

3. Moissonneuse-batteuse (2) selon la revendication 1 ou 2, **caractérisée en ce que** le piston (18) peut être déplacé au moyen d'un actionneur électrique.

4. Moissonneuse-batteuse (2) selon l'une quelconque des revendications précédentes, **caractérisée en ce que** le capteur d'humidité des grains (12) est disposé dans un carter de transition (15) de la trémie à grains (10) en aval d'une chicane (14), qui est agencée de telle sorte que, en cours de service, elle dévie vers la cellule (13) le flux de grains distribué par l'élévateur (4).

Fig. 1A

*Fig. 1B*

*Fig. 1C*

*Fig.2*

*Fig.3*

*Fig. 4*

*Fig. 5*

Fig. 6A

FÜLLSTROM 250

TIEFPASS FILTER 230

Mischer LO SIG 224

HOCHPASS FILTER 223

1/N 222

U_M 246
ZELL U. REF.-STRÖME

TIEFPASS FILTER 228

Mischer LO SIG 220

HOCHPASS FILTER 219

Σ 1/N 218

W_M 242
ZELL- U. REF. - SPANNUNGEN
} ZUM MICROCONTROLLER

TIEFPASS FILTER 226

Mischer LO SIG 216

HOCHPASS FILTER 215

Σ 214

I_R1 I_R2 I_C I_F

V_R1 V_R2 V_C

*Fig.6B*

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- EP 0804872 A
- EP 0908086 A
- US 5327708 A